Europäisches Patentamt

European Patent Office

Office européen des brevets

(11) Publication number: **0 469 084 B1**

# EUROPEAN PATENT SPECIFICATION

(45) Date of publication of patent specification: **05.04.95** (51) Int. Cl.⁶: **C07K 14/62**, A61K 38/28

(21) Application number: **90908000.4**

(22) Date of filing: **17.04.90**

(86) International application number:
**PCT/US90/02070**

(87) International publication number:
**WO 90/12814 (01.11.90 90/25)**

(54) **HEPATOSPECIFIC INSULIN ANALOGUES.**

(30) Priority: **20.04.89 US 340929**

(43) Date of publication of application:
**05.02.92 Bulletin 92/06**

(45) Publication of the grant of the patent:
**05.04.95 Bulletin 95/14**

(84) Designated Contracting States:
**AT BE CH DE FR GB IT LI LU NL SE**

(56) References cited:
**EP-A- 214 826**

**CHEMICAL ABSTRACTS, vol. 94, no. 7, 16 February 1981, Columbus, Ohio, USA, Danho, W. Et al.: "A14-Phenyalanine-insulin: a synthetic analog." page 623; column 2; ref. No. 47733X, see abstract**

**PROCEEDING OF THE NATIONAL ACADEMY OF SCIENCES OF USA, vol. 86, no. 2, January 89, WASHINGTON US pages 458 - 461; Schwartz, G.P. et al.: "A highly patent insulin: DES-(B26-B30)-(AspB10, Tyr B25-NH2)**

**insulin (human)", see abstract**

(73) Proprietor: **MOUNT SINAI SCHOOL OF MEDI-CINE OF THE CITY UNIVERSITY OF NEW YORK**
**One Gustave L. Levy Place**
**New York, NY 10029 (US)**

(72) Inventor: **KATSOYANNIS, Panayotis, G.**
**69 Drake Lane**
**Manhasset, NY 11030 (US)**

(74) Representative: **Lucas, Brian Ronald**
**Lucas & Co.**
**135 Westhall Road**
**Warlingham**
**Surrey CR6 9HJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

Background of the Invention

The present invention relates to novel insulin analogues and their use in pharmaceutical compositions for the treatment of diabetes.

Insulin is a hormone which has a key role in the regulation of growth and metabolism in vertebrates. Severe metabolic derangements occur in the absence of insulin resulting from the failure of many cells to utilize glucose and amino acids normally. The inability to metabolize glucose leads in man to diabetes mellitus, a complex chronic metabolic disorder in which there is abnormal carbohydrate, fat and protein metabolism. In its most fully expressed clinical form, diabetes mellitus is characterized by an absolute or relative deficiency of insulin or insulin activity and is associated with glucosuria, ketonuria, growth arrest, and negative nitrogen balance. These conditions can ultimately lead to death from acute metabolic acidosis caused by unrestrained oxidation of fatty acids or inanition which results from the lack of sufficient lipid reserves needed to generate ketone bodies. Inanition is defined as a condition characterized by marked weakness, extreme weight loss, and a decrease in metabolism resulting from prolonged and severe insufficiency of food. Dorland's Illustrated Medical Dictionary, 25th Edition.

The discovery and purification of insulin in the 1920's and its association with diabetes mellitus provided the means to intervene in the disease. See, e.g., Bliss, The Discovery of Insulin (1983), University of Chicago Press, Chicago, Ill. Today, insulin administration to diabetic patients is the primary therapeutic means for controlling the disease.

Insulin is a ca 6000 dalton polypeptide which is composed of two short peptide chains, termed A and B, which are linked to each other by invariant disulfide bridges. In almost all insulins studied, the A chain, which is 21 amino acids long, also contains an internal disulfide bridge. The B chain is 30 amino acids in length. Like many eukaryotic proteins, insulin is synthesized in a precursor form which is post-synthetically processed to the mature two polypeptide chain active hormone.

The immediate precursor of insulin is proinsulin, a single chain polypeptide composed of the B and A chains linked to a connecting peptide of approximately 31 amino acids, termed the C-peptide, by adjacent pairs of basic residues. The order of the three peptides in the proinsulin molecule is $NH_2$-B chain-Arg-Arg-C-peptide-Lys-Arg-A chain-COOH. The translation product of insulin mRNA, however, is preproinsulin which is proinsulin that contains at its $NH_2$ terminus a 24 amino acid largely hydrophobic signal peptide characteristic of proteins that are either transported through or inserted into cellular membranes.

Preproinsulin is synthesized in pancreatic beta cells located within the islets of Langerhans which are dispersed throughout the pancreas. Removal of the signal peptide occurs in the rough endoplasmic reticulum with the resulting fully folded oxidized proinsulin being transported to the Golgi apparatus for packaging into secretion granules. The folded proinsulin is stabilized by disulfide bonds. During maturation of the secretion granules, the folded proinsulin molecule is cleaved by specific proteases at the paired basic residues to liberate insulin and the C-peptide.

As discussed above, therapy for diabetes mellitus includes administration of controlled amounts of insulin to the diabetic patient. The insulin so administrated has, for the most part, been obtained from animal pancreases, notably bovine and porcine. Bovine and porcine insulins function to maintain hormonal homeostasis in the same way as human insulin with about the same potency but, because they are foreign proteins, can elicit an immunological response which diminishes their usefulness. More recently, human insulin, generated by recombinant DNA techniques, has been added to the therapeutic armamentarium. The use of human insulin, produced by recombinant DNA or other techniques, is not likely to produce the adverse immunological problems attendant the use of animal insulins. Even with the availability of natural human insulin, however, administration of the hormone to diabetics has not been always sufficient to restore normal metabolism. There is thus a need for alternative insulins with better activity or other means of therapy for diabetes.

U.S. Patent 4,992,417 discloses a superactive human insulin analogue, [10-Aspartic Acid-B] human insulin, which has increased activity over natural human insulin. Specifically, [10-Aspartic Acid-B] human insulin was determined to be 4 to 5 times more potent than natural insulins. U.S. Patent No. 4,992,417 and International Application Serial No. PCT/US88/02289 disclose other superactive insulin analogues, des-pentapeptide (B26-B30)-[Asp$^{B10}$,Tyr$^{B25}$-α-carboxamide] human insulin, (B26-B30)-[Glu$^{B10}$, Tyr$^{B25}$-α-carboxamide] human insulin, and further insulin analogs of the formula des(B26-B30)-[X$^{B10}$, Tyr$^{B25}$-α-carboxamide] human insulin, in which X is a residue substituted at position 10 of the B chain. These insulin analogues have potencies anywhere from 11 to 20 times that of natural human insulin. All of the above-described insulin analogues involve amino acid substitutions along the A or B chains of natural human insulin, which increase the potency of the compound or change other properties of the compound.

None of the current insulin delivery routes of natural insulin and known insulin analogues exactly mimic insulin secretion from the normal pancreas. Normally, insulin enters the splanchnic venous circulation, thereby exposing the liver to higher concentrations of insulin than those to which peripheral tissue is exposed. With standard subcutaneous administration of insulin, plasma glucose concentrations may be normalized, but glucose recycling and the production and utilization of protein and lipids may not be. In addition, peripheral vascular tissues are exposed to higher than normal insulin concentrations. Although the long-term effects of these metabolic abnormalities remains to be defined, there is considerable evidence that peripheral hyperinsulinemia may be a significant risk factor for the development of atherosclerosis.

Hepatospecific insulin analogues, or those which are more active in the liver than in adipose tissue, offer several advantages over currently available insulin therapy. Using such analogues it may be possible to obtain preferential hepatic uptake during peripheral subcutaneous administration, thereby mimicking more closely the metabolic balance between the liver and peripheral tissues. Although attempts to mimic this pattern with intraperitoneal injection of insulin have been undertaken, this technique has the potential disadvantages of difficultly for intraperitoneal access and risk of peritonitis. Hepatospecific insulin achieves the same effect as intraperitoneal insulin, without these increased risks.

Summary of the invention

New insulin analogues have now been synthesized and found to be hepatospecific. These insulin analogues have the A13 and/or A14 amino acid in the A chain substituted (replaced) by tryptophan. Alternatively, the A14 amino acid can be substituted by another hydrophobic amino acid, but tryptophan or other bulky amino acids are preferred. Phenylalanine is excluded: W. Danho et al., Chemical Abstracts 94: 47733x (Proc. Intl. Symp., 2nd 1979 [pub. 1980]) disclose a porcine A14-phenylalanine insulin which has virtually the same biological activity as native insulin.

The invention also relates to pharmaceutical compositions for the treatment of diabetes in patients needing such treatment which comprise a therapeutically effective amount of an insulin analogue according to the claimed invention, together with a pharmaceutically acceptable carrier.

Furthermore, the invention relates to the claimed insulin analogues and pharmaceutical compositions for use in treating diabetes, and to their use in the preparation of a formulation for use in treating diabetes.

Brief description of the Drawings

Figure 1 is a chromatogram showing the elution of crude sheep [Trp$^{14}$] A chain S-sulfonate from a Cellex-E column (200-350 ml of effluent).

Figure 2A is a chromatogram showing the elution of a mixture of sheep B chain S-sulfonate and sheep [Trp$^{14}$] A chain S-sulfonate at the initial chromatographic separation from a CM-cellulose column.

Figure 2B is a reversed-phase HPLC chromatogram showing the rechromatography of the material in the peak shown in Figure 2A (140-180 ml of effluent).

Figure 3 is graph comparing the stimulation of lipogenesis in rat adipocytes by natural insulin and [Trp$^{14}$-A] insulin.

Figure 4 is a graph comparing the inhibition of gluconeogenesis in FAO cells in which inhibition of glucose secretion is determined as a function of natural insulin or [Trp$^{14}$] insulin.

Description of the Preferred Embodiments

The A14 amino acid is replaced by an amino acid residue selected from the group consisting of tryptophan, naphthylalanine, $N^{\gamma}$-dansyl-$\alpha$, $\gamma$-diaminobutyric acid, leucine, valine, phenylalanine, and other hydrophobic amino acids.

The preferred insulin analogue, [Trp$^{14}$-A] insulin, has the formula

```
        _____
       |                   |                    |
       |                 ┌──┬──┐─────────S-S─────────┐
       |                 │  │  │                    │
       | H-Gly-Ile-Val-Glu-Gln-Cys-Cys-Thr-Ser-Leu-Cys-Ser-
       S
       S
       | Leu-Trp-Gln-Leu-Glu-Asn-Tyr-Cys-Asn-OH
       |                               |
       |                               |_____
       |
       |_____
       |                           |
       H-Phe-Val-Asn-Gln-His-Leu-Cys-Gly-Ser-His-Leu-Val-Glu-Ala-Leu-
                                                                      S
                                                                      S
       Tyr-Leu-Val-Cys-Gly-Glu-Arg-Gly-Phe-Phe-Tyr-Thr-Pro-Lys-Thr-OH  |
                       |                                                 |
                       |_____|
```

The term insulin analogue refers to a protein which has the basic A chain and B chain structure of human (and other species of) insulin and contains all of the half cysteine residues in the same position as present in native insulin. Thus, insulin analogues retain the disulfide bridge arrangement of natural insulins. Useful insulin analogues can differ from native insulins by the addition, deletion, substitution or modification of one or more amino acids in one or both chains of the molecule, but must retain at least some portion of insulin potency. See e.g. Katsoyannis, Treatment of Early Diabetics (1979), pp. 319-327, Plenum Publ. Corp.; Blondell, Adv. Prot. Chem. (1972), vol. 26, pp. 330-362.

The insulin analogues of the invention, which differ from natural insulin by the substitutions of tryptophan for the naturally-occurring amino acid at position A14 were unexpectedly found to have higher potency in hepatic cells than in adipose tissue.

The most important property of these insulin analogues is their hepatospecificity, whereby they are more active in the liver than in the adipose tissue or peripheral tissue. For example, in adipose tissue [Trp$^{14}$-A] insulin displays an activity or about 60% of that of natural insulin (lipogenesis assay) whereas in the liver exhibits an activity (in terms of inhibiting gluconeogenesis) of 90% of that of natural insulin. It should be noted that whereas insulin stimulates the utilization of glucose in the peripheral tissue (i.e., adipose tissue), in the liver insulin inhibits the de novo synthesis of glucose (inhibition of gluconeogenesis).

It is believed that substitution of the A14 amino acid residue in the molecule of insulin with other bulky residues such as naphthylalanine or N$^{\gamma}$-dansyl-$\alpha,\gamma$-diaminobutyric acid or other hydrophobic amino acid residues (except phenylalanine) such as leucine or valine also leads to hepatospecific insulin analogues.

Modifications of the B chain moieties of the above mentioned insulin analogues at the B10 and B25 positions may be made in accordance with applicant's previous applications, U.S. Patent 4,992,417, the disclosure of which is incorporated herein by reference. These additional substitutions yield superactive insulins believed to be hepatospecific.

The claimed insulin analogues can be produced by any of a variety of techniques known to those skilled in the art. For example, the component A and B chains of the insulin analogues can be synthesized by any of the known peptide synthesis techniques, including solid phase peptide synthesis techniques and solution techniques, e.g. fragment condensation. See, e.g. Erickson and Merrifield, The Proteins (1976), vol. 2, chapter 3, Academic Press, New York; Blake et al. Proc. Natl. Acad. Sci. (1983), vol. 80, pp. 1556-1559 for a discussion of peptide synthesis techniques. Some of the claimed insulin analogues can also be prepared by combining human or sheep B chain, isolated following reduction or oxidative sulfitolysis of intact pancreatic or recombinant insulin, with a modified A chain prepared by peptide synthetic techniques or recombinant DNA methods.

Recombinant DNA methods for producing the insulin A or B chains having substituted naturally-occurring amino acids at any position include, but are not limited to, cloning and expression of an in vitro

4

synthesized DNA which codes for such an A or B chain amino acid sequence. Alternatively, an organism, such as bacteria, which expresses human insulin A or B chain could be induced to produce a modified chain by any of the techniques of in vitro site-directed mutagenesis. See e.g. Smith, Ann. Rev. Genet. (1985), vol. 19, pp. 423-463; Botstein et al. Science (1985), vol. 229, pp. 1193-1201.

In general, to prepare insulin having a modified A chain, sheep insulin B chains, obtained by any known technique are combined with the modified A chains prepared by any convenient technique. The B and modified A chains are preferably in their stabilized S-sulfonated forms which can then be recombined by known procedures to form the intact active insulin analogues. Known recombination techniques are taught by U.S. Patent No. 3,420,810 to Katsoyannis and U.S. Patent No. 4,421,685 to Chance et al. U.S. Patent No. 4,421,685 provides a single step process for forming an insulin which involves bringing together an S-sulfonated A chain and S-sulfonated B chain in the presence or a thiol reducing agent, such as dithiothreitol or cysteine, in an aqueous medium. The conditions for recombination include (1) a pH of about 10.5, (2) a total protein concentration of about 0.1 to 50 mg/ml, and (3) a thiol reducing agent in a concentration which produces about 0.4 to 2.5 SH groups per each - $S-SO_3$ group present in the total A and B chain S-sulfonates present in the mixture. The formation of the insulin analogues occurs by maintaining the reaction at a temperature of about 0 to 5°C in an environment which provides a source of oxygen to allow the formation of the insulin S-S bonds.

Once the recombination reaction has been completed, the insulin analogue can be isolated and assayed for purity and activity by a variety or techniques known to those skilled in the art. Commonly employed techniques for purification of insulin and insulin analogues are chromatographic techniques, such as high performance liquid chromatography (HPLC), gel filtration and ion-exchange chromatography. Purity of the product can be determined by a variety of techniques including inter alia HPLC, polyacrylamide gelelectrophoresis, amino acid analysis and amino acid sequencing.

Although insulin analogues, in general, maintain some residual insulin activity, the potency of such analogues is usually only a fraction of that of natural insulins. The potency of human, bovine and porcine insulins in USP standards is about 25-26 IU (international units) per mg protein. Standard assay for measuring insulin potency include inter alia (1) insulin radioreceptorassays, in which the relative potency of an insulin is defined as the ratio of insulin to insulin analogue required to displace 50% of $^{125}$I-insulin specifically bound to insulin receptors present on cell membranes, e.g. a rat liver plasma membrane fraction, isolated rat adipocytes, or isolated rat hepatocytes; (2) lipogenesis assays, performed e.g. with rat adipocytes, in which relative insulin potency is defined as the ratio of insulin to insulin analogue required to achieve 50% of the maximum conversion of [3-$^3$H] glucose into organic-extractable material (i.e. lipids); (3) glucose oxidation assays in isolated fat cells in which the relative potency of the insulin analogue is defined as the ratio of insulin to insulin analogue to achieve 50% of the maximum conversion of glucose-1-[$^{14}$C] into [$^{14}CO_2$]; (4) insulin radioimmunoassays which can determine the immunogenicity of insulin analogues by measuring the effectiveness by which insulin or an insulin analogue competes with $^{125}$I-insulin in binding to specific anti-insulin antibodies; (5) inhibition of gluconeogenesis performed on confluent monolayers of a well differentiated hepatoma cell line (FAO) in which inhibition of glucose secretion into the medium is determined as a function of the concentration of insulin or insulin analogue. The relative potency of the analogue is defined as the ratio of the concentration of insulin to analogue required to produce half-maximal inhibition of glucose production; and (6) other assays which measure the binding of insulin or an insulin analogue to cells, such as cultured lymphocytes, known to possess specific insulin receptors.

The insulin analogues of the invention may also be formulated into pharmaceutical compositions for administration to diabetic patients. The pharmaceutical compositions comprise an insulin analogue according to the claimed invention in an amount which is therapeutically effective (which is preferably defined as effective in promoting the attainment of hormonal homeostasis in the diabetic patient), together with a pharmaceutically acceptable carrier. As with all insulin preparations for treatment of diabetes, adequate therapeutic amounts of the active compound to achieve hormonal homeostasis in individual patients must be determined. Factors to be considered include the severity of the diabetic condition and the route of administration of the composition. Ultimately the particular physician treating the diabetic patient has discretion in the amount of the pharmaceutical composition and route of administration. Natural insulins are generally given to a patient in a therapeutic dosage to afford about 0.02 to about 5 units of human insulin activity per kilogram body weight per day. See e.g. U.S. Patent No. 4,652,547.

Pharmaceutical compositions containing a therapeutically effective amount of an insulin analogue according to the claimed invention may be administered parenterally to a diabetic patient in need of insulin treatment. Preferably the composition is administered intramuscularly, subcutaneously or intravenously. The composition may also be administered to the patient by nasal spray. Alternatively, for long-term controlled homeostasis, the composition may be incorporated into an implantable pump for administration to the

EP 0 469 084 B1

patient. Such implantable devices which provide a controlled dosage of drug to a patient over a long period of time are known in the art. The composition additionally comprises a pharmaceutically acceptable carrier which must not be deleterious to the patient. The carrier must also not have any adverse effect on the active component of the composition, i.e., [Trp$^{14}$-A) insulin or another insulin analogue of the invention. Suitable carriers and other additives for pharmaceutical compositions which contain therapeutically effective amounts of the claimed insulin analogues as the active component may be found in U.S. Patent No. 4,652,547 which provides for insulin-containing compositions.

As an example, the synthesis of [Trp$^{14}$-A] insulin was achieved by the interaction of S-sulfonated sheep B chain with S-sulfonated forms of [Trp$^{14}$] A chain (XX, below) of sheep insulin. The procedures described by Katsoyannis et al., Biochemistry, 6:2635-2642 (1967) and Chance et al., Pept. Proc. Am. Pept. Symp. 7th, 721-728 (1981) were followed. The synthesis of the A chain analogue involved, as the key step, the construction of the protected heneicosapeptide (XII below), containing the entire amino acid sequence of the respective A chain. For the construction of the protected heneicosapeptide either solid phase methodology according to Merrifield et al. J. Am. Chem. Soc., 85, 2149-2154 (1963) and Merrifield et al., Biochemistry, 21, 5020-5031 (1982) or the fragment condensation approach was employed. The synthesis by the latter approach involved the coupling of the C-terminal pentapeptide (sequence A$^{17}$-A$^{21}$) with the adjacent pentapeptide (sequence A$^{12}$-A$^{16}$) to produce the C-terminal decapeptide (sequence A$^{12}$-A$^{21}$). The latter compound was coupled with the adjacent tripeptide (sequence A$^{9}$-A$^{11}$) to yield the C-terminal tridecapepide (sequence A$^{9}$-A$^{21}$), which in turn was coupled with the adjacent tetrapeptide (sequence A$^{5}$-A$^{8}$) to produce the C-terminal heptadecapeptide (sequence A$^{5}$-A$^{21}$). The final coupling step involved the coupling of the C-terminal heptadecapeptide with the N-terminal tetrapeptide to yield the protected heneicosapeptide (XII or XIX, below). The usual blocking groups were employed for the protection of the secondary functions of the various amino acids (i.e., Bzl for Ser, Glu, Asn and Tyr and PMB for Cys) with the exception of the indole function of Trp, which was protected with the Mts group in accordance with Fujii et al., Chem. Pharm. Bull. (Japan) 32, 2660-2665 (1984). Removal of the blocking groups from the protected heneicosapeptide ether with 1M trifluoromethanesulfonic acid - thioanisole in TFA containing m-cresole and EDT according to Yajima and Fujii, J. Am. Chem. Soc., 103, 5867-5871 (1981) and Fujii et al., supra, using the modification described recently by Ogawa et al., J. Protein Chem., 3, 327-348 (1984) or by the low/high hydrogen fluoride procedure according to Tam et al., J. Am Chem. Soc., 105, 6442-6455 (1983) and sulfitolysis of the resulting reduced products yielded the S-sulfonated chain analogue XX.

The invention is further illustrated by the following specific examples which are not intended in any way to limit the scope of the invention.

Example 1

Synthesis of Sheep [Trp$^{14}$-A] Insulin

A. Synthesis of A Chain

Boc-Gln-Leu•OMe (I)

To a solution of H•Leu•OMe, 11.3 g of HCl in DMF (50 mL) was added along with 9.1 mL of TEA followed by 17.6g of Boc-Gln•ONp. After 24 hours the mixture was filtered and the filtrate concentrated to a small volume, under reduced pressure, and diluted with 500 mL of AcOEt and 100 mL water. The organic layer was washed with 1 N NH$_4$OH, water, 0.5 N HCl and water, dried and concentrated to dryness. The residue solidified on trituration with ether - petroleum ether and crystallized from ethyl acetate - ether: wt: 13.7 g (76.6%); mp: 126°C; $[\alpha]_D^{25}$ -35.3° (c l, methanol). Anal. Calcd. for C$_{17}$H$_{31}$N$_3$O$_6$: C, 54.7; H, 8.37; N, 11.3. Found: C, 55.0; H, 8.26; N, 11.3.

Z(OMe)-Trp(Mts)-Gln-Leu•OMe (II)

A solution of compound I (3.73 g) in TFA - anisole (8 mL - 2 mL) was stored at room temperature for 1.5 hours and concentrated to dryness under reduced pressure. The residue was triturated with a mixture of ether - petroleum ether (1:2, v/v) and dried over KOH in vacuo. A solution of this material in 20 mL of DMF containing 2.8 mL of TEA was cooled to 0°C and was added to 6.21 g of the azide prepared from Z(OMe)-Trp-(Mts)-NHNH$_2$ according to Fujii et al., supra, following the method of Ogawa et al., supra. The following reagents were used in this reaction: DMF (20 mL), 6.3 N HCl in DMF (3.5 mL), tert-butyl nitrate (1.5 mL) and TEA (3.1 mL). After 40 hours at 4°C the mixture was diluted with AcOEt (600 mL) and saturated NaCl

6

(100 mL). The organic layer was washed with 10% citric acid and saturated NaCl, dried and concentrated to dryness. Upon trituration with ether the residue solidified and then crystallized from ethanol: wt: 3.6 g (45%); mp: 171-173 °C; $[\alpha]_D^{25}$ -41.4° (c I, DMF). Anal. Calcd. for $C_{41}H_{51}N_5O_{10}S$: C, 61.1; H, 6.4; N, 8.7. Found: C, 61.0; H, 6.5; N, 8.5.

## Boc-Leu-Trp(Mts)-Gln-Leu•OMe (III)

A solution of compound II (2.0 g) in a mixture of TFA - anisole - EDT (10 mL - 1 mL - 0.3 mL) was stored at 0 °C for 30 minutes and at room temperature for 30 minutes and then processed as described in the synthesis of compound II. 1.7 g of Boc-Leu•ONp was added to a solution of the resulting $N^\alpha$-deprotected peptide salt in DMF (35 mL) containing TEA (0.6 mL) cooled to 0 °C. After 24 hours at room temperature the mixture was diluted with 600 mL of AcOEt, washed successively with 1 N $NH_4OH$, saturated NaCl, 0.5 N HCl and saturated NaCl, dried and concentrated to a small volume. The precipitated product was collected and recrystallized from AcOEt: wt: 1.51 g (71%); mp: 188-190 °C; $[\alpha]_D^{25}$ -20.6° (c I, DMF). Anal. Calcd. for $C_{43}H_{62}N_6O_{10}S$: C, 60.4; H, 7.3; N, 9.8. Found: C, 60.1; H, 7.5; N. 9.8. Amino acid analysis after 4 M MSA hydrolysis gave the ratios: $Glu_{1.0}Leu_{2.0}Trp_{0.8}$.

## Boc-Ser(Bzl)-Leu-Trp(Mts)-Gln-Leu•OMe (IV)

Deblocking of compound III (4.46 g) with TFA - anisole - EDT (15 mL - 1.2 mL - 0.8 mL) and isolation of the resulting $N^\alpha$-deprotected peptide salt was carried out as described in the synthesis of compound III. 2.07g of Boc-Ser(Bzl)•OH was added at 0 °C to a solution of this material in DMF (60 mL) containing TEA (1.1 mL), followed by addition of 1.65 g of N, N'-dicyclohexylcarbodiimide and 1.08 g of 1-hydroxybenzotriazole. After 24 hours at room temperature the urea by-product was filtered off and the filtrate diluted with 600 mL AcOEt and saturated with 100 mL of NaCl. The organic layer was washed as usual, dried and concentrated to a small volume. The precipitated product was collected and reprecipitated from ethyl acetate - ether: wt: 4.58 g (85%); mp: 187-188 °C; $[\alpha]_D^{25}$ -21.6° (c I, DMF). Anal. Calcd. for $C_{53}H_{73}N_7O_{12}S$: C, 61.7; H, 7.1; N, 9.5. Found: C, 61.4; H, 7.2; N, 9.3. Amino Acid analysis after 4 M MSA hydrolysis gave the ratios: $Ser_{1.0}Glu_{1.0}Leu_{2.0}Trp_{0.7}$.

## Boc-Ser(Bzl)-Leu-Trp(Mts)-Gln-Leu-NHNH$_2$ (V)

A solution of compound IV (4.2 g) in a mixture of DMF (35 mL) and ethanol (10 mL) containing hydrazine hydrate (1.0 mL) was stored at room temperature for 24 hours and then diluted with 200 mL of 50% aqueous ethanol. The precipitate product was collected and reprecipitated from methanol - water: wt: 4.0 g (94%); mp: 203-205 °C; $[\alpha]_D^{25}$ -6.1° (c I, DMSO). Anal. Calcd. for $C_{52}H_{73}N_9O_{11}S$: C, 60.5; H, 7.1; N, 12.2. Found: C, 60.9; H, 7.4; N, 12.0. A 4 M MSA hydrolysate gave the ratios: $Ser_{0.9}Glu_{1.0}Leu_{2.0}Trp_{0.7}$.

## Boc-Tyr(Bzl)-Cys(PMB)-Asn•OBzl (VI)

A solution of 7.39 g of Boc-Cys-(PMB)-Asn•OBzl prepared according to Ohta et al., J. Protein Chem., 7, 55-65 (1988) in TFA - anisole (15 mL - 3 mL) was stored at O °C for 30 minutes and at room temperature for 1.5 hours and then concentrated under reduced pressure. The residue was triturated with a mixture of ether and petroleum ether (1:1 v/v) and dried over KOH in vacuo. 7g of Boc-Tyr(Bzl)•ONp was added to a solution of this material in DMF (60 mL) containing TEA (2.3 mL) and cooled to 0 °C. After 24 hours at room temperature the mixture was diluted with 600 mL of AcOEt and 100 mL of 1 N $NH_4OH$. The organic phase was washed (1 N $NH_4OH$, 0.5 N HCl and water), dried and concentrated to a small volume. The precipitated product was collected and recrystallized from AcOEt: wt: 8.7 g (84%); mp: 174-175 °C; $[\alpha]_D^{25}$ -18.6° (c I, DMF). Anal. Calcd. for $C_{43}H_{50}N_4O_9S$: C, 64.6; H, 6.30; N, 7.0. Found: C, 64.0; H, 6.35; N, 7.0.

## Boc-Asn-Tyr(Bzl)-Cys(PMB)-Asn•OBzl (VII)

8.2 g of compound VI was deblocked with TFA - anisole (22 mL - 2.2 mL) as described above. Trituration of the residue with ether caused the precipitation of the $N^\alpha$-deprotected peptide salt which was filtered off and dried over KOH in vacuo. 3.9 g of Boc-Asn•ONp was added to a solution of this product in DMF (50 mL) containing TEA (1.8 mL) and cooled to 0 °C. After 24 hours at room temperature the reaction mixture was diluted with 200 mL of methanol and the precipitated product was filtered off, washed (1 N $NH_4OH$, 0.5 N HCl and water), dried and reprecipitated from DMF - methanol: wt: 6 g (64%); mp: 237-238 °C; $[\alpha]_D^{25}$ -37.4 ° (c l, DMF). Anal. Calcd. for $C_{47}H_{56}N_6O_{11}S$: C, 61.8; H, 6.18; N, 9.2. Found: C, 61.6; H, 6.30; N, 9.1.

### Boc-Glu(OBzl)-Asn-Tyr(Bzl)-Cys(PMB)-Asn•OBzl (VIII)

The deblocking of compound VII (5.5 g) and isolation of the $N^\alpha$-deprotected peptide salt was carried out as described in the synthesis of compound VII. 3.3 g of Boc-Glu-(OBzl)•ONp (prepared from the method of Sandrin et al., Helv. Chim. Acta, 46: 1637-1669 (1963)) was added to a solution of this product in DMF (50 mL) containing TEA (0.85 mL) at 0 °C. The reaction mixture was processed as in the synthesis of compound VII: wt: 5.6 g (81%); mp: 206-207 °C;
$[\alpha]_D^{25}$ -41.4 ° (c l, DMF). Anal. Calcd. for $C_{59}H_{69}N_7O_{14}S$: C, 62.6; H, 6.14; N, 8.7. Found: C, 62.3; H, 5.94; N, 8.5. Amino acid ratios in a 6 N HCl hydrolysate: $Asp_{1.8}Glu_{1.0}Tyr_{1.0}$. Cys(PMB) was not determined.

### Boc-Ser(Bzl)-Leu-Trp(Mts)-Gln-Leu-Glu(OBzl)-Asn-Tyr(Bzl)-Cys(PMB)-Asn•OBzl (IX)

The deblocking of compound VIII (2.3 g) and isolation of the $N^\alpha$-deprotected peptide salt was carried out as described in the synthesis of compound VII. 2.98 g of the azide prepared from compound V (according to Ogawa et al., supra) was added to a solution of this product in DMF (35 mL) containing TEA (0.42 mL) and cooled to 0 °C. The reagents used for this reaction were as follows: DMF (20 mL), 6.3 N HCl in DMF (0.92 mL), tert-butyl nitrite (0.42 mL) and TEA (0.98 mL). After 48 hours at 4 °C the reaction mixture was diluted with AcOEt (20 mL) and 5% citric acid (100 mL) and the precipitated product was filtered off, washed with water and methanol and reprecipitated from DMF - methanol: wt: 1.55 g (38%), mp: 245-246 °C;
$[\alpha]_D^{25}$ -15.7 ° (c l, DMSO). Anal. Calcd. for $C_{106}H_{131}N_{14}O_{23}S_2•2H_2O$: C, 61.5; H, 6.6; N, 9.5. Found: C, 61.4; H, 6.5; N, 9.7. Amino acid radios in a 6 N HCl hydrolysate:
$Asp_{2.0}Ser_{0.9}Glu_{2.1}Leu_{2.1}Tyr_{1.0}$. Trp and Cys(PMB) were not determined.

### Boc-Gly-Val-Cys(PMB)-Ser(Bzl)-Leu-Trp(Mts)-Gln-Leu-Glu(OBzl)-Asn-Tyr(Bzl)-Cys(PMB)-Asn•OBzl (X)

A solution of compound IX (0.95 g) in TFA - anisole - EDT (5 mL - 0.11 mL - 0.08 mL) was stored at 0 °C for 30 minutes and at room temperature for 1.5 hours. The excess TFA was removed by evaporation under reduced pressure and the residue triturated with ether. The precipitate formed was filtered off and dried over KOH In vacuo. 0.82 g of the azide prepared from Boc-Gly-Val-Cys(PMB)-$NHNH_2$ according to Ogawa et al., supra, was added to a solution of this product in DMF (25 mL) containing TEA (0.09 mL). The reagents used for this reaction were was follows: DMF (10 mL), 6.3 N HCl in DMF (0.51 mL), tert-butyl nitrite (0.24 mL) and TEA (0.49 mL). After 48 hours at 4 °C the reaction mixture was diluted with AcOEt (20 mL) and 5% citric acid (60 mL) and the precipitated product was collected and reprecipitated from DMF - methanol: wt: 0.93 g (82%); mp: 267-268 °C; $[\alpha]_D^{25}$ -17.4 ° (c l, DMSO).
Anal. Calcd. for $C_{124}H_{156}N_{17}O_{27}S_3.3H_2O$: C, 60.4; H, 6.6; N, 9.7. Found: C, 60.3; H, 6.4; N, 9.9. Amino acid ratios in a 6 N HCl hydrolysate: $Asp_{1.9}Ser_{0.9}Glu_{2.0}Gly_{1.0}Val_{1.0}Leu_{2.1}Tyr_{0.9}$. Trp and Cys (PMB) were not determined.

### Boc-Gln-Cys(PMB)-Cys(PMB)-Ala-Gly-Val-Cys(PMB)-Ser(Bzl)-Leu-Trp(Mts)-Gln-Leu-Glu(OBzl)-Asn-Tyr(Bzl)-Cys(PMB)-Asn•OBzl (XI)

The deblocking of compound X (0.82 g) with TFA - anisole - EDT (5 mL - 0.5 mL - 0.1 mL) and the isolation of the $N^\alpha$-deprotected peptide salt was carried out as described above. 1.06 g of the azide prepared from Boc-Gln-Cys(PMB)-Cys(PMB)-Ala-$NHNH_2$ according to Ogawa et al., supra, was added to a solution of this product in DMF (40 mL) containing TEA (0.1 mL). The reagents used for this reaction were as follows: DMF (20 mL), 6.3 N HCl in DMF (0.43 mL), tert-butyl nitrite (0.21 mL) and TEA (0.42 mL). After 48 hours at 4 °C the reaction mixture was diluted with 200 mL of methanol and the precipitated heptadecapeptide derivative was collected and reprecipitated from DMF - methanol: wt: 0.85 g; mp: >270 °C; $[\alpha]_D^{25}$ -21.4 ° (c l, DMSO). Amino acid ratios in a 6 N HCl hydrolysate are as follows: $Asp_{2.0}Ser_{0.9}Glu_{3.1}Gly_{1.0}Ala_{1.2}Val_{0.9}Leu_{1.9}Tyr_{0.7}$. Trp and Cys(PMB) were not determined.

Z-Gly-Ile-Val-Glu(OBuᵗ)-Gln-Cys(PMB)-Cys(PMB)-Ala-Gly-Val-Cys(PMB)-Ser(Bzl)-Leu-Trp(Mts)-Gln-Leu-Glu-(OBzl)-Asn-Tyr(Bzl)-Cys(PMB)-Asn•OBzl (XII)

The deblocking of compound XI (0.6 g) with TFA - anisole - EDT (6 mL - 0.6 mL - 0.1 mL) and the isolation of the resulting product was carried out as described above. 0.49 g of the azide prepared from Z-Gly-Ile-Val-Glu(OBuᵗ)-NHNH$_2$ (prepared with the method of Katsoyannis et al., J. Am. Chem. Soc., 88:5622-5625 (1966) Was added to a solution of this material in DMF (40 mL) containing TEA (0.06 mL). The reagents used for this reaction were as follows: DMF (20 mL), 6.3 N HCl in DMF (0.25 mL), tert-butyl nitrite (0.11 mL) and TEA (0.3 mL). After 48 hours at 4°C, the reaction mixture was diluted with methanol (200 mL) and the precipitated protected heneicosepeptide was collected and reprecipitated from DMF - methanol: wt: 0.53 g (76%); mp: >270°C. Amino acid analysis after 6 N HCl-hydrolysis gave the following molar ratios: Asp$_{2.0}$Ser$_{0.9}$Glu$_{4.0}$Gly$_{2.1}$Ala$_{1.0}$ Val$_{1.5}$Ile$_{0.6}$Leu$_{1.8}$Tyr$_{0.7}$. Trp and Cys(PMB) were not determined.

B. Synthesis of S-Sulfonated [Trp¹⁴] A Chain (XX)

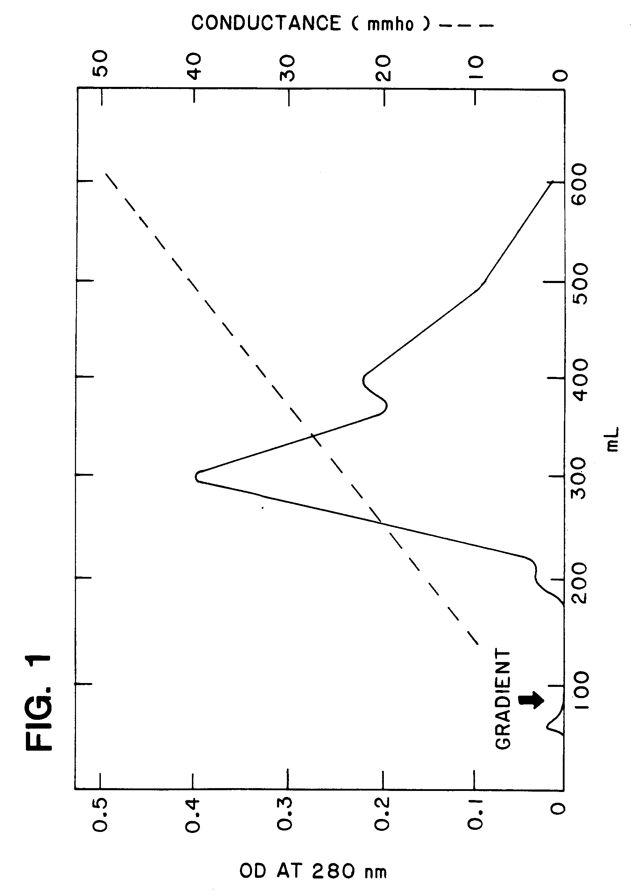

The protected heneicosapeptide XII (200 mg) was treated with 1 M trifluoromethanesulfonic acid in TFA (4.3 mL) containing thioanisole (0.66 mL), m-cresole (0.59 mL) and EDT (0.47 mL) at 0° for 2 hours. This solution was then cooled to -10°C, and 8 M guanidine hydrochloride (25 mL) containing concentrated NH$_4$OH (5 mL) was added dropwise and with vigorous stirring. During this process, the temperature of the mixture was kept below 5°C. The resulting mixture (pH ~ 5) was extracted 3 times with ether (50 mL each), and to the aqueous layer, adjusted to pH 8.9 with NH$_4$OH, were added 1.2g of sodium sulfite and 0.6g of sodium tetrathionate. The mixture was stirred at room temperature for 3.5 hours, then placed in Spectrapor membrane tubing No. 3 and dialyzed against four changes of distilled water (4 L each) at 4°C for 24 hours. Lyophilization of the dialysate afforded the crude S-sulfonated chain analogue which was dissolved in 6 mL of 0.015 M NH$_4$HCO$_3$ and chromatographed on a Sephadex G-15 column (4.2 x 45cm), equilibrated and eluted with 0.015M NH$_4$HCO$_3$. The effluent corresponding to the main peak, as monitored with an ISCO spectrophotometer, was lyophilized and the sheep insulin [Trp¹⁴] A chain S-sulfonate was obtained as a white powder: wt: 130.3 mg. For purification, this material (71.3 mg) was dissolved in 0.1 M Tris-HCL buffer (pH 7.0; 3 mL) and applied on a Cellex-E column (1.2 x 43 cm), which was equilibrated with the same buffer. Elution of the column was carried out with Tris-HCl buffer (pH 7.0) and a linear NaCl gradient as described previously by Chu et al., Biochemistry, 26, 6966-6971 (1987). The chromatographic pattern, as monitored with an ISCO spectrophotometer and a conductivity meter (Radiometer, Copenhagen), is shown in Figure 1. The effluent corresponding to the main peak (230-360 mL) was collected, dialyzed as above and lyophilized: Wt: 35.4 mg.

Amino acid analysis of the synthetic chain analogue was performed in two acid hydrolysates; one using 6 N HCl and another using 4 M MSA (for Trp determination) as in the method of Simpson et al., J. Biol. Chem., 251, 1936-1940 (1976). The amino acid composition, expressed in molar ratios, was in agreement with the theoretically expected values. Digestion of the synthetic chain analogue with aminopeptidase M and amino acid analysis of the digest gave the expected molar ratios. The synthetic material was completely digested by the enzyme indicating that the stereochemical purity of the constituent amino acids was preserved during the synthetic processes.

C. Synthesis and Isolation of Sheep [Trp¹⁴-A] Insulin

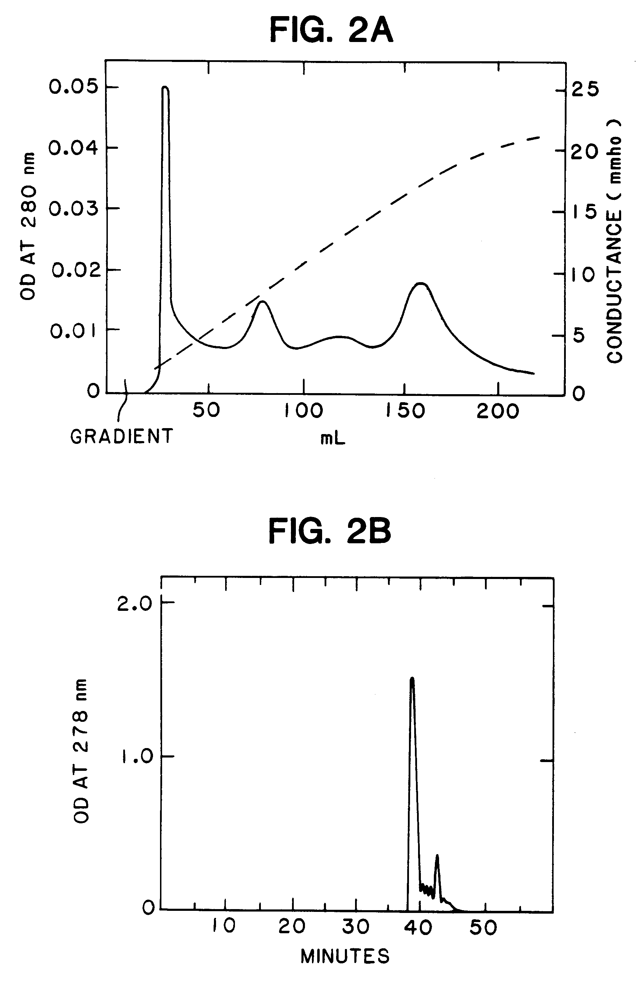

The synthesis of sheep [Trp¹⁴-A] insulin was carried out by the interaction of the S-sulfonated [Trp¹⁴] sheep A chain (XX) with the S-sulfonated sheep (which is the same as bovine) B chain in the presence of dithiothreitol following the method of Chance et al., supra. A solution of sheep B chain S-sulfonate (10 mg) (prepared as described by Katsoyannis et al., Biochemistry, 6, 2635-2642 (1967)), [Trp¹⁴] A chain S-sulfonate (20 mg) and dithiothreitol (6.83 mg) in 0.1 M glycine buffer (pH 10.5, 6 mL) was stirred at 4°C for 24 hours and then processed as described previously by Katsoyannis et al., Biochemistry, 6, 2656-2668 (1967). Isolation and purification of the insulin analogue from the combination mixture were carried out by chromatography on a CM-cellulose column (0.9 x 24 cm) with an acetate buffer (Na⁺, 0.024 M, pH 3.3) and an exponential NaCl gradient as described by Katsoyannis et al., supra. The elution pattern obtained, as monitored by an ISCO spectrophotometer and a conductivity meter (Radiometer, Copenhagen), is shown in Figure 2A. 1.3mg of the [Trp¹⁴-A] insulin was isolated from the effluent (145-175 mL) via picrate as the hydrochloride in accordance with Katsoyannis et. al., supra. Final purification of this material was accomplished by reversed-phase HPLC on a Vydac® 218 TP column (0.45 x 25 cm) connected to an LKB liquid chromatography system. Chromatography was carried out at a flow rate of 0.5 mL/min with a 10-50% linear gradient of 2-propanol in 0.1% TFA over 60 minutes (Fig. 2B). Lyophilization of the effluent under the main peak achieved the sheep [Trp[14]-A] insulin in a highly purified form.

Amino acid analysis of the synthetic material, after 6N HCl hydrolysis, gave a composition expressed in molar ratios in good agreement with the theoretically expected values.

Example 2

Analysis of [Trp[14]-A] Insulin Potency And Binding Properties

In this Example the materials used and the analytical procedures followed are those found in Kitagawa et al., Biochemistry, 23: 1405-1413 (1984). $^{125}$I-insulin for receptor binding studies and [3-$^3$H] glucose for lipogenesis were obtained from DuPont NEN Research Products. Cellulose acetate membrane filters, 0.2 $\mu$m pore size, were products of Sartorius. Glass-fiber filters, Type GFC, were from Whatman. Collagenase, Type II crude, was obtained from Worthington. The scintillation fluids Filtron-X®, Hydrofluor® and Soluscint-O® were products of National Diagnostics. Crystalline bovine insulin was from Sigma, and fatty-acid free bovine serum albumin from Boehringer - Mannheim Biochemicals.

A. Insulin Receptor Binding Assay

The ability of [Trp[14]-A] insulin to inhibit the specific binding of $^{125}$I-insulin to insulin receptors was examined in a fraction of rat liver enriched in plasma membranes, isolated rat adipocytes, and isolated rat hepatocytes. The first two procedures were done in accordance with Burke et. al, Biochemistry 19:4547-4556, (1980).

Briefly, triplicate 0.2ml incubations contained $^{125}$I-insulin, $3 \times 10^{-10}$M, unlabelled insulin or [Trp[14]-A] insulin prepared as in Example 1, and plasma membranes (20-40 $\mu$g of protein) in 0.1 M sodium phosphate, pH 7.4, containing 0.6% fraction V bovine serum albumin. Following incubation for 45 min at 24°C, the mixtures were diluted with 2.0 ml of ice cold 0.1 M sodium phosphate, pH 7.4, containing 0.1% fraction V bovine serum albumin and immediately filtered through cellulose-acetate filters. The filters were washed twice with the ice cold buffer, dried, and then radioactivity was counted in a scintillation counter using Filtron-X®. Relative potency was obtained as the concentration ratio of unlabelled insulin to [Trp[14]-A] insulin required to inhibit 50% of the specific binding of $^{125}$I-insulin to the receptor preparation. In this assay [Trp[14]-A] insulin displayed a potency of about 60% of that of the natural hormone.

B. Lipogenesis Assay

These assays measured the ability of the insulin analogue as compared to bovine insulin to convert [3-$^3$H] glucose into lipids.

Adipocytes were prepared by incubating epididymal and perirenal fat pads obtained from male rats weighing 200-300 g with 1.0 mg/ml collagenase for 60 min at 37°C, followed by filtration through gauze and then through fine-mesh silk. Cells were washed twice by flotation in a clinical centrifuge before suspension for use. The incubation medium was Krebs-Ringer bicarbonate containing half the recommended calcium, 0.5 mM glucose, and 3% fatty acid free bovine serum albumin, with 95% $O_2$-5% $CO_2$ as the gas phase. Triplicate lipogenesis incubations contained 1.0 ml of adipocyte suspension (20-40 mg dry wt cells) and bovine insulin or [Trp[14]-A] insulin, prepared as in Example 1. Cells were preincubated for 45 min at 37°C before the addition of [3-$^3$H] glucose. Incubation was continued for 60 min and stopped by the addition of 0.2 ml of 5 N $H_2SO_4$ and 0.2 ml of corn oil to aid in the extraction of lipids. Samples were extracted with 10 ml of Soluscint-0® for 30 min at room temperature before counting the radioactivity in a scintillation counter. Under these conditions, [3-$^3$H] glucose was not extracted into the organic phase containing the scintillation fluors and was essentially uncounted. Zero and 100% stimulation of lipogenesis were defined as radioactivity observed in the absence and presence, respectively, of $9.1 \times 10^{-10}$M insulin (5.5 ng/ml). Relative potency was obtained as the concentration ratio of insulin to [Trp[14]-A] insulin required to produce 50% of the maximum stimulation of lipogenesis.

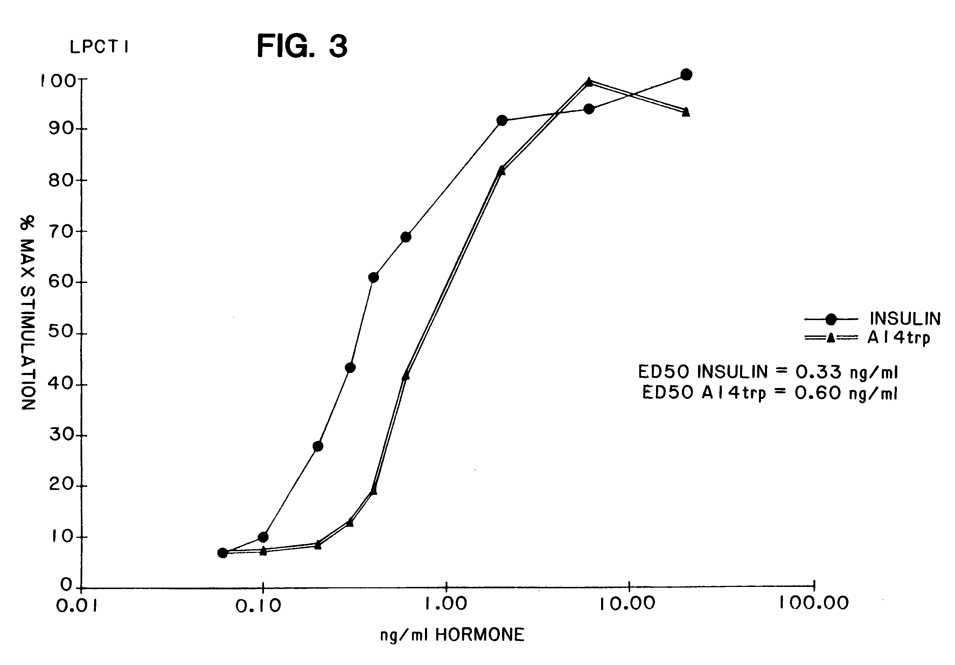

Figure 3 shows the stimulation of [3-$^3$H] glucose into organic-extractable material (i.e., lipids) in isolated rat adipocytes by [Trp[14]-A] insulin ( = ▲ = ) as prepared in Example 1 and bovine insulin (-●-). Stimulation, expressed a percent of maximum, was plotted as a function of the agonist concentration. The data points represent the mean triplicate determinations in representative assays performed four times.

For both insulins the same maximum stimulation of lipogenesis was observed and half-maximal stimulation was produced by about twice the concentration of agonist. The potency of the synthetic insulin in lipogenesis (approximately 60% compared to the natural hormone) thus reflects its behavior in receptor binding assays employing adipocytes.

C. Inhibition of Gluconeogenesis Assay

This assay was carried out on confluent monolayers of a well differentiated hepatoma cell line (Lauris et al. Endocrinology 118:2519-2524 (1986)) in which glucose production was monitored with a commercial glucose oxidase kit (Sigma kit no. 510-A, Sigma Technical Bulletin 510, 1983). Inhibition of glucose secretion into the medium was determined as a function of the concentration of insulin or insulin analogue, and the relative potency of the analogue was defined as the ratio of the concentration of insulin to analogue required to produce half-maximal inhibition of glucose production. The cell culture and glucose production was carried out as described by Lauris et al., supra.

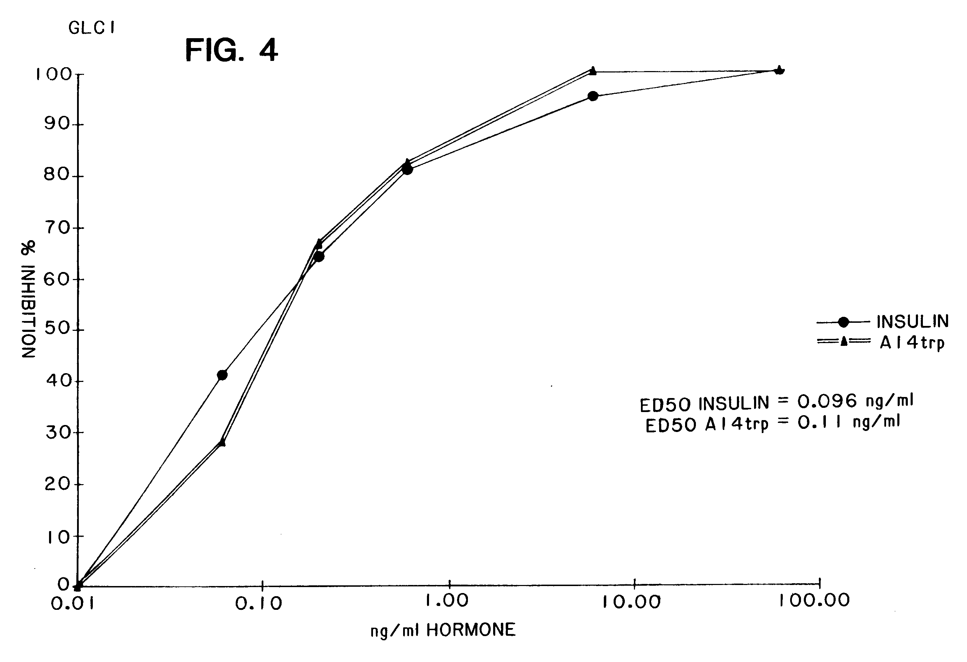

Figure 4 shows the inhibition of gluconeogenesis in FAO cells, expressed as a per cent of maximum, and is presented as a function of insulin (-●-) and [Trp [14]-A] insulin (= ▲ =) concentration. For both insulins the same maximum inhibition of gluconeogenesis was observed and half-maximal inhibition was produced by approximately the same concentration of agonist. The potency of the synthetic insulin in inhibiting gluconeogenesis is calculated to be about 90% relative to the natural hormone.

**Claims**

1. An insulin analogue having an A chain comprising amino acids A1 to A21 and a B chain comprising amino acids B1 to B30, in which the A14 amino acid is replaced by a tryptophan, naphthylalanine, N$^\gamma$-dansyl-$\alpha$, $\gamma$-diaminobutyric acid, leucine or valine residue or the residue of another hydrophobic amino acid except phenylalanine.

2. An insulin analogue having an A chain comprising amino acids A1 to A21 and a B chain comprising amino acids B1 to B30, in which the A13 and/or the A14 amino acid is replaced by a tryptophan residue.

3. An insulin analogue according to Claim 1 having the formula

H-Gly-Ile-Val-Glu-Gln-Cys-Cys-Thr-Ser-Leu-Cys-Ser-

Leu-Trp-Gln-Leu-Glu-Asn-Tyr-Cys-Asn-OH

H-Phe-Val-Asn-Gln-His-Leu-Cys-Gly-Ser-His-Leu-Val-Glu-Ala-Leu-

Tyr-Leu-Val-Cys-Gly-Glu-Arg-Gly-Phe-Phe-Tyr-Thr-Pro-Lys-Thr-OH

4. A pharmaceutical composition suitable for the treatment of diabetes in a patient in need of such treatment comprising a therapeutically effective amount of an insulin analogue according to Claim 1, 2 or 3, together with a pharmaceutically acceptable carrier.

5. A pharmaceutical composition according to Claim 4 in a form suitable for intramuscular administration.

6. A pharmaceutical composition according to Claim 4 in a form suitable for subcutaneous administration.

7. A pharmaceutical composition according to claim 4 in a form suitable for intravenous administration.

8. A pharmaceutical composition according to Claim 4 in a form suitable for administration by implantable pump.

9. A pharmaceutical composition according to Claim 4 in a form suitable for administration by nasal spray.

10. An insulin analogue according to Claim 1, 2 or 3, or a composition according to any one of Claims 4 to 9, for use in treating diabetes.

11. A composition according to Claim 5, 6, 7 or 8, for use in treating diabetes by intramuscular, subcutaneous or intravenous administration or by administration from an implantable pump.

12. An implantable pump incorporating an insulin analogue claimed in Claim 1, 2 or 3 or a composition claimed in Claim 8.

13. Use of an insulin analogue claimed in Claim 1, 2 or 3, or a composition claimed in any one of Claims 4 to 9, for the preparation of a formulation for treating diabetes.

**Patentansprüche**

1. Insulinanalogon mit einer A-Kette, die die Aminosäuren A1 bis A21 und eine B-Kette, die die Aminosäuren B1 bis B30 umfaßt, in welche die A14 Aminosäure durch Tryptophan, Naphthylalanin, $N^{\gamma}$-Dansyl-$\alpha,\gamma$-Diaminobytyric-Säure, Leucin oder Valinrest oder der Rest einer anderen Hydrochobic-Aminosäure ausgetauscht wird, außer Phenylalanin.

2. Insulinanalogon mit einer A-Kette, die die Aminosäuren A1 bis A21 und eine B-Kette, die die Aminoäsuren B1 bis B30 umfaßt, in welche die A13 und/oder die A14 Aminosäure durch einen Tryptophanrest ausgetauscht wird.

3. Insulinanalogon nach Anspruch 1 mit der Formel

12

4.  Pharmazeutische Zusammensetzung, geeignet für die Behandlung von Diabetes bei einem Patienten, die eine therapeutisch wirksame Menge eines Insulinanalogons nach Anspruch 1, 2 oder 3 zusammen mit einem pharmazeutisch annehmbaren Träger enthält.

5.  Pharmazeutische Zusammensetzung nach Anspruch 4 in einer geeigneten Form zur intramuskulären Verabreichung.

6.  Pharmazeutische Zusammensetzung nach Anspruch 4 in einer geeigneten Form zur subkutanen Verabreichung.

7.  Pharmazeutische Zusammensetzung nach Anspruch 4 zur intravenösen Verabreichung.

8.  Pharmazeutische Zusammensetzung nach Anspruch 4 zur Verabreichung durch eine implantierbare Pumpe.

9.  Pharmazeutische Zusammensetzung nach Anspruch 4 zur Verabreichung durch ein Nasenspray.

10. Insulinanalogon nach Anspruch 1, 2 oder 3 oder eine Zusammensetzung nach einem der Ansprüche 4 bis 9 zur Anwendung bei der Behandlung von Diabetes.

11. Zusammensetzung nach Anspruch 5, 6, 7 oder 8 zur Anwendung bei der Behandlung von Diabetes durch intramuskuläre, subkutane oder intravenöse Verabreichung oder durch Verabreichung durch eine implantierbare Pumpe.

12. Implantierbare Pumpe, die ein Insulinanalogon enthält, nach Anspruch 1, 2 oder 3 oder eine Zusammensetzung nach Anspruch 8.

13. Anwendung eines Insulinanalogons nach Anspruch 1, 2 oder 3 oder einer Zusammensetzung nach einem der Ansprüche 4 bis 9 zur Preparation einer Mischung für die Behandlung von Diabetes.

**Revendications**

1.  Analogue d'insuline ayant une chaîne A comprenant des acides aminés A1 à A21 et une chaîne B comprenant des acides aminés B1 à B30, dans lequel l'acide aminé A14 est remplacé par du tryptophane, de la naphtylalanine, du $N^\gamma$-dansyl-$\alpha$, de l'acide $\gamma$-diaminobutyrique, un résidu valine ou leucine ou le résidu d'un autre acide aminé hydrophobe, excepté de la phénylalanine.

2.  Analogue d'insuline ayant une chaîne A comprenant des acides aminés A1 à A21 et une chaîne B comprenant des acides aminés B1 à B30, dans lequel l'acide aminé A13 et/ou A14 est remplacé par un résidu tryptophane.

EP 0 469 084 B1

3. Analogue d'insuline selon la revendication 1 répondant à la formule

4. Composition pharmaceutique appropriée pour le traitement du diabète chez un patient nécessitant ce traitement comprenant une quantité thérapeutiquement efficace d'un analogue d'insuline selon la revendication 1, 2 ou 3, ainsi qu'un véhicule pharmaceutiquement acceptable.

5. Composition pharmaceutique selon la revendication 4 sous une forme appropriée pour l'administration par voie intramusculaire.

6. Composition pharmaceutique selon la revendication 4 sous une forme appropriée pour l'administration par voie sous-cutanée.

7. Composition pharmaceutique selon la revendication 4 sous une forme appropriée pour l'administration par voie intraveineuse.

8. Composition pharmaceutique selon la revendication 4 sous une forme appropriée pour l'administration par pompe pouvant être implantée.

9. Composition pharmaceutique selon la revendication 4 sous une forme appropriée pour l'administration par spray nasal.

10. Analogue d'insuline selon la revendication 1, 2 ou 3, ou composition selon l'une quelconque des revendications 4 à 9, pour l'utilisation dans le traitement du diabète.

11. Composition selon la revendication 5, 6, 7 ou 8, pour l'utilisation dans le traitement du diabète par administration par voie intramusculaire, sous-cutanée ou intraveineuse ou par administration à partir d'une pompe pouvant être implantée.

12. Pompe pouvant être implantée incorporant un analogue d'insuline revendiqué dans la revendication 1, 2 ou 3 ou une composition revendiquée dans la revendication 8.

13. Utilisation d'un analogue d'insuline revendiqué dans la revendication 1, 2 ou 3, ou d'une composition revendiquée dans l'une quelconque des revendications 4 à 9, pour la préparation d'une formulation pour le traitement du diabète.

14

FIG. 1

# FIG. 2A

# FIG. 2B

FIG. 3

GLC I

## FIG. 4

% INHIBITION

ng/ml HORMONE

● INSULIN
▲ A14trp

ED50 INSULIN = 0.096 ng/ml
ED50 A14trp = 0.11 ng/ml

EP 0 469 084 B1